# EUROPÄISCHE PATENTSCHRIFT

(11) **EP 2 764 783 B1**
(45) Veröffentlichungstag und Bekanntmachung des Hinweises auf die Patenterteilung: **03.10.2018**
(21) Anmeldenummer: 14153696.1
(22) Anmeldetag: 03.02.2014
(51) Int. Cl.: A24F 47/00

(54) **Elektronische Zigarette oder Pfeife**
Electronic cigarette or pipe
Cigarette ou pipe électronique

(30) Priorität: 11.02.2013 DE 202013100606 U
(43) Veröffentlichungstag der Anmeldung: 13.08.2014
(73) Patentinhaber: EWWK UG, 72072 Tübingen (DE)
(72) Erfinder: Hodapp, Georg, 72622 Nürtingen (DE); Nothacker, Steffen, 72072 Tübingen (DE)
(74) Vertreter: Ruckh, Rainer Gerhard

(56) Entgegenhaltungen:
- EP-A1- 2 319 334
- EP-A1- 2 468 116
- US-A1- 2011 277 757
- TheEcigStop: "Apex Alpha UltraLight .mp4 - YouTube", , 22. Oktober 2011 (2011-10-22), XP054975402, Gefunden im Internet: URL:http://www.youtube.com/watch?v=f2ehwbf KATI [gefunden am 2014-05-13]

## Beschreibung

Die Erfindung betrifft eine elektronische Zigarette oder Pfeife.

Eine derartige elektronische Zigarette oder Pfeife umfasst generell ein Mundstück und einen Verdampfer, der im Wesentlichen einen Liquidspeicher, einen Liquidträger und ein Heizelement aufweist. Im Liquidspeicher vorhandenes Liquid wird selbsttätig über eine Kapillarwirkung des Liquidträgers dem Heizelement zugeführt. Das Heizelement ist dabei in Kontakt mit diesem Liquidträger. Die elektronische Zigarette oder Pfeife weist weiterhin ein Aktivierungsmittel, insbesondere einen Schalter wie einen Drucktaster, Unterdruckschalter oder Luftzugschalter auf, mit welchem ein Benutzer einen Inhalationsvorgang starten kann. Durch die Betätigung des Aktivierungsmittels wird das Heizelement aktiviert und verdampft im Liquidträger vorhandenes Liquid. Der dadurch entstehende Dampf wird dann vom Benutzer inhaliert.

Derartige elektronische Zigaretten und Pfeifen werden verstärkt als Alternativen zu Tabakzigaretten und Tabakpfeifen eingesetzt, da sie diesen gegenüber gesundheitliche Vorteile aufweisen. Dieser gesundheitliche Vorteil wird dadurch erhalten, dass bei der Verdampfung des Liquids, das typischerweise aus einer Trägerflüssigkeit aus Propylenglykol oder Glycerin, Wasser, Aromastoffen und gegebenenfalls Nikotin besteht, keine krebserregende Stoffe erzeugt werden.

Bei bekannten elektronischen Zigaretten oder Pfeifen werden Liquidträger, bestehend aus Glasfasern oder oxidierten Edelstahlsieben eingesetzt, die aufgrund ihrer Kapillarwirkung Liquid aus dem Liquidspeicher in den Liquidträger fördern.

Ein wesentlicher Nachteil bei aus Glasfasern bestehenden Liquidträgern besteht darin, dass diese Glasfasern leicht brechen und dass entstehende kleine Glasfaserbruchstücke vom Benutzer eingeatmet werden können, welche dann zu lungengängigen, gesundheitsschädlichen Partikeln führen können.

Derartige Nachteile bestehen beim Einsatz von oxidierten Edelstahlsieben zur Ausbildung von Liquidträgern zwar nicht. Jedoch besteht dort die Gefahr, dass bei Reinigungsvorgängen die elektrisch isolierende Oxidschicht auf dem Edelstahlsieb abgebaut wird, so dass dann ein Kurzschluss zwischen dem Edelstahlsieb und dem Heizelement entsteht.

Ein weiterer wesentlicher Nachteil von Liquidträgern, die aus Glasfasern oder auch aus Edelstahlsieben gebildet sind, besteht darin, dass sich diese bei Berühren leicht verformen. Dies führt zu starken Veränderungen der Verdampfungseigenschaften, da sich durch die Verformungen des Liquidträgers dessen Zuordnung zu den umgebenden Heizelementen verändert. Damit ergeben sich unerwünscht hohe Toleranzen der Verdampfungsleistungen der elektronischen Zigarette oder Pfeife selbst innerhalb einer Produktionsserie.

Die EP 2 468 116 A1 betrifft eine elektronische Zigarette mit einer aus einem Bündel von Fibern bestehenden Kapillarkörper, mit dem Flüssigkeit aus einem Flüssigkeitsspeicher gefördert wird. In geringem Abstand zum Kapillarkörper ist ein Heizelement zum Verdampfen von Flüssigkeit in dem Kapillarkörper vorgesehen. Der Kapillarkörper kann aus Keramik-basierten Materialien bestehen.

Der Erfindung liegt die Aufgabe zugrunde, eine elektronische Zigarette oder Pfeife mit reproduzierbarer hoher Funktionalität bereitzustellen.

Zur Lösung dieser Aufgabe sind die Merkmale des Anspruchs 1 vorgesehen. Vorteilhafte Ausführungsformen und zweckmäßige Weiterbildungen der Erfindung sind in den Unteransprüchen beschrieben.

Die Erfindung betrifft eine elektronische Zigarette oder Pfeife mit einem Mundstück und einem Verdampfer und umfasst einen Liquidträger und ein Heizelement, welches mit einem Aktivierungsmittel aktivierbar ist. Das aktivierte Heizelement verdampft im Liquidträger vorhandenes Liquid. Der Liquidträger ist nur von einem aus einem Sinterwerkstoff bestehenden Formteil gebildet. Das Heizelement ist direkt in Kontakt mit einer Oberfläche des Liquidträgers. Der Liquidträger ist rohrförmig, insbesondere als Hohlzylinder mit kreisförmigem Querschnitt ausgebildet. Das Heizelement liegt an der inneren Mantelfläche des rohrförmigen Liquidträgers an, wobei der Liquidspeicher den rohrförmigen Liquidträger umschließt und an dessen äußerer Mantelfläche anliegt.

Mit dem Begriff elektronische Zigarette oder Pfeife sind auch Zigaretten oder Zigarren umfasst. Weiterhin kann die Erfindung auch für Shishas und ähnliche Einheiten eingesetzt werden.

Durch die erfindungsgemäße Verwendung eines Sinterwerkstoffs zur Ausbildung des Liquidträgers wird auf überraschend einfache Weise die Funktionalität der elektronischen Zigarette oder Pfeife signifikant erhöht.

Der aus dem Sinterwerkstoff aufgebaute Liquidträger weist eine hohe thermische und mechanische Stabilität auf, wobei der so ausgebildete Liquidträger eine hohe Maßgenauigkeit aufweist und somit insbesondere auch bei einer Serienfertigung toleranzarm und reproduzierbar herstellbar ist.

Der Liquidträger besteht erfindungsgemäß aus nur einem Formteil bestehend aus einem Sinterwerkstoff, welches fertigungstechnisch kostengünstig herstellbar ist und dabei einfach an die konstruktiven Gegebenheiten der elektronischen Zigarette oder Pfeife angepasst werden kann. Prinzipiell könnte der Liquidträger auch aus mehreren Formteilen bestehen.

Generell kann der Liquidträger aus einem porösen Festkörper bestehen, das heißt die Erfindung ist nicht auf Liquidträger bestehend aus Sinterwerkstoffen begrenzt.

Ein wesentlicher Vorteil des erfindungsgemäßen Liquidträgers besteht weiterhin darin, dass dieser eine hohe Stabilität, Maßhaltigkeit und vor allem eine hohe Formbeständigkeit aufweist. Insbesondere ist eine unerwünschte Verformung des Liquidträgers bei Reinigungsvorgängen oder einem Sturz der elektronischen Zigarette oder Pfeife auf einen harten Untergrund ausgeschlossen.

Damit wird, im Gegensatz zu Liquidträgern, die aus Glasfasern oder Edelstahlsieben bestehen, eine hochgenaue Reproduzierbarkeit der Verdampfungseigenschaften des Verdampfers erhalten. Dies beruht darauf, dass durch die Formstabilität des aus einem Sinterwerkstoff bestehenden Liquidträgers dessen räumliche Zuordnung zu dem Heizelement exakt erhalten bleibt. Insbesondere ist ausgeschlossen, dass ein auf dem Liquidträger aufliegendes Heizelement durch Verformungen des Liquidträgers abgehoben wird, das heißt es ist ausgeschlossen, dass sich Luftbrücken zwischen dem Heizelement und dem Liquidträger bilden.

Derartige Luftbrücken würden nicht nur die Verdampfungsleistungen beeinträchtigen. Vielmehr käme es dadurch zu einem Aufglühen des beispielsweise aus einem Heizdraht bestehenden Heizelements, was zu einem verbrannten Liquidgeschmack führen würde.

Eine reproduzierbare und gleichbleibend hohe Verdampfungsleistung der elektronischen Zigarette oder Pfeife wird auch deshalb erhalten, da bei dem den Liquidträger bildenden Sinterwerkstoff die Kapillarwirkung fertigungstechnisch exakt vorgegeben werden kann.

Die zur Ausbildung des Liquidträgers eingesetzten Sinterwerkstoffe weisen eine hohe Beständigkeit gegen Säuren, Laugen, Aromastoffe und Liquidgrundstoffe auf. Die so ausgebildeten Liquidträger können somit auch gut gereinigt werden, wobei sogar auch Säuren oder Laugen für die Reinigungsvorgänge eingesetzt werden können. Auch mechanische Reinigungen mittels Bürsten oder in einem Ultraschallbad sind möglich. Dies stellt einen wesentlichen Vorteil gegenüber herkömmlichen Liquidträgern dar, da beispielsweise Liquidträger aus Glasfasern nicht mechanisch gereinigt werden können, da sie ansonsten brechen würden. Bei Liquidträgern aus oxidierten Edelstahlsieben besteht das Problem, dass bei chemischen oder mechanischen Reinigungsvorgängen die Oxidschicht abgelöst wird.

Die aus Sinterwerkstoff bestehenden Liquidträger sind weiterhin auch physiologisch und allgemein gesundheitlich unbedenklich, insbesondere auch deshalb, da anders als bei Liquidträgern aus Glasfasern keine lungengängige Partikel abgelöst werden.

Prinzipiell können zur Ausbildung des Liquidträgers der erfindungsgemäßen elektronischen Zigarette oder Pfeife elektrisch leitfähige Sinterwerkstoffe verwendet werden. Um Kurzschlüsse mit aufliegenden Heizelementen zu vermeiden, müssen dann diese elektronisch leitfähigen Sinterwerkstoffe mit einer isolierenden, das heißt elektrisch nicht leitfähigen Schicht überzogen werden.

Um den Aufwand eines Aufbringens einer solchen Isolierschicht zu vermeiden, besteht der Liquidträger der erfindungsgemäßen elektronischen Zigarette oder Pfeife vorteilhaft aus einem elektrisch nicht leitfähigen Sinterwerkstoff.

Insbesondere kann der Sinterwerkstoff von einer gesinterten Keramik gebildet sein.

Weiterhin kann der Sinterwerkstoff von Borosilikatglas Aluminiumoxid-Mullit, Cordierit, Siliziumnitrit, Siliziumcarbid, Tialit, Steatit, Porzellan, Zirkon oder ZrO₂ gebildet sein.

Da der erfindungsgemäße Liquidträger vorteilhaft aus nur einem Formteil besteht kann dieser rationell und kostengünstig hergestellt werden. Zudem kann die Geometrie auch bei seriengefertigten Formteilen exakt vorgegeben und an die weiteren Komponenten der elektronischen Zigarette oder Pfeife angepasst sein. Beispielsweise können die aus dem Sinterwerkstoff bestehenden Formteile platten-, stift- oder rohrförmig ausgebildet sein.

Gemäß einer besonders vorteilhaften Ausführungsform weist die erfindungsgemäße elektronische Zigarette oder Pfeife einen Liquidspeicher auf, aus welchem dem Liquidträger Liquid zugeführt wird. Alternativ sind auch Ausgestaltungen ohne einen solchen Liquidspeicher möglich. Bei derartigen Ausführungsformen handelt es sich um sogenannte Tröpfel- beziehungsweise Dripper-Verdampfer.

Besonders zweckmäßig ist das Heizelement direkt in Kontakt mit einer Oberfläche des Liquidträgers.

Mit dem aus dem Sinterwerkstoff bestehenden Liquidträger wird eine hohe thermische und mechanische Stabilität auch bei mechanischen Belastungen erhalten, da sich der erfindungsgemäße Liquidträger bei Auftreten von mechanischen Kräften nicht verformt. Die Kontaktflächen zwischen dem Liquidträger und dem Heizelement bleiben somit über große Zeiträume erhalten, was zu einer konstanten Verdampfungsleistung der elektronischen Zigarette oder Pfeife führt.

Als Heizelemente werden besonders vorteilhaft Heizdrähte eingesetzt, die kostengünstig herstellbar sind und weiterhin auch exakt an Oberflächen der Leitträger angebracht werden können.

Ebenso kann das Heizelement aus bandförmigem elektrisch leitendem Material bestehen.

Weiterhin kann das Heizelement als leitfähige Beschichtung auf einer Oberfläche des Liquidträgers ausgebildet sein.

Die Erfindung wird im Folgenden anhand der Zeichnungen erläutert. Es zeigen:
- Figur 1:: Ausführungsbeispiel der erfindungsgemäßen elektronischen Zigarette.
- Figur 2:: Variante eines Verdampfers der elektronischen Zigarette gemäß Figur 1 (nicht zur Erfindung gehörend)
- Figur 3:: Draufsicht auf einen Liquidträger des Verdampfers gemäß Figur 2 mit zugeordnetem Heizelement.
- Figur 4:: Erfindungsgemäße Ausbildung eines Verdampfers der elektronischen Zigarette gemäß Figur 1.

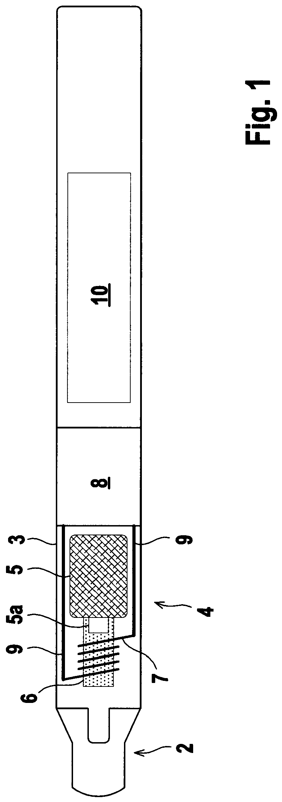

Figur 1 zeigt ein Ausführungsbeispiel einer elektronischen Zigarette 1. Die elektronische Zigarette 1 weist ein Mundstück 2 auf. Das Mundstück 2 ist Bestandteil eines Gehäuses 3, das zweckmäßig an die Form herkömmlicher TabakZigaretten angepasst ist. In dem an das Mundstück 2 anschließenden Gehäuseteil sind die Komponenten eines Verdampfers 4 integriert, nämlich ein Liquidspeicher 5, ein Liquidträger 6 und ein Heizelement 7. Der Liquidspeicher 5 enthält ein Liquid, welches aus einer Trägerflüssigkeit aus Propylenglykol oder Glycerin sowie Wasser besteht und weiterhin Aromastoffe sowie gegebenenfalls Nikotin enthält. Der Liquidträger 6 ist an den Liquidspeicher 5 gekoppelt, so dass mittels Kapillarwirkung Liquid aus dem Liquidspeicher 5 in den Liquidträger 6 geführt ist. Das Heizelement 7 ist in Kontakt mit dem Liquidträger 6. In dem an den Verdampfer 4 anschließenden Gehäuseteil ist eine elektronische Baueinheit 8 integriert. Die elektronische Baueinheit 8 umfasst ein nicht gesondert dargestelltes Aktivierungsmittel zur Aktivierung des Heizelements 7. Das Aktivierungsmittel kann von einem mikroprozessor-gesteuerten Schalter, wie einem Drucktaster, einem Unterdruckschalter oder Luftzugschalter gebildet sein. Alternativ kann als Aktivierungsmittel eine sensorgesteuerte Einheit vorgesehen sein, die die Zugstärke eines von einem Benutzer ausgeübten Zugs an der elektronischen Zigarette erfasst und in Abhängigkeit hiervon die Verdampfungsleistung des Verdampfers 4 vorgibt. Die Komponenten der elektronischen Baueinheit 8 sind über elektrische Anschlussmittel mit dem Verdampfer 4 elektrisch verbunden. Figur 1 zeigt als Beispiel dieser elektrischen Anschlussmittel Anschlussklemmen 9, mittels derer das Heizelement 7 mit der elektronischen Baueinheit 8 verbunden ist. Im hinteren Bereich des Gehäuses 3 befindet sich eine autarke Energieversorgung, die im vorliegenden Fall von einer Batterie 10 gebildet ist. Alternativ kann ein Akkumulator vorgesehen sein. Alternativ kann anstelle einer elektronischen Zigarette 1 auch eine elektronische Pfeife mit einem entsprechenden Aufbau vorgesehen sein.

Die Funktionsweise der elektronischen Zigarette ist derart, dass durch den Benutzer das Aktivierungsmittel aktiviert wird um einen Inhalationsvorgang auszulösen

Die Aktivierung erfolgt durch manuelles Betätigen des Aktivierungsmittels in Form eines Schalters oder durch ein Ausüben eines Zugs am Mundstück 2 mit einer bestimmten Zugstärke. Durch ein solches Betätigen des Aktivierungsmittels wird das Heizelement 7 aktiviert und verdampft Liquid im Liquidträger 6. Der entstehende Dampf wird dann vom Benutzer inhaliert.
Der Liquidträger 6 besteht erfindungsgemäß aus einem Sinterwerkstoff, wobei im vorliegenden Fall der Liquidträger 6 aus nur einem Formteil, bestehend aus einem solchen Sinterwerkstoff, gebildet ist.
Zur Ausbildung des Liquidträgers 6 wird ein nicht leitfähiger Sinterwerkstoff verwendet, im vorliegenden Fall eine gesinterte Keramik. Alternativ ist der Sinterwerkstoff von Borosilikatglas gebildet.
Der Liquidträger 6 besteht im vorliegenden Fall aus einem stiftförmigen, kreiszylinderischen, massiven Formteil aus Sinterwerkstoff. Der so gebildete Liquidträger 6 sitzt an einem Anschlussstück 5a des als kreiszylinderischen Tanks ausgebildeten Liquidspeichers 5 auf. Dabei fällt die Längsachse des Liquidträgers 6 mit der Längsachse des Liquidspeichers 5 zusammen, das heißt die beiden Elemente sind koaxial angeordnet.
Das Heizelement 7 liegt an der Mantelfläche des Liquidträgers 6 dicht an, so dass keine Lufteinschlüsse zwischen Liquidträger 6 und Heizelement 7 vorhanden sind. Das Heizelement 7 besteht im vorliegenden Fall aus einem Heizdraht. Alternativ können auch bandförmige Heizelemente 7 vorgesehen sein. Weiterhin kann auch eine leitfähige Beschichtung auf der Mantelfläche des Liquidträges als Heizelement 7 vorgesehen sein.
Durch die Kapillarwirkung des den Liquidträger 6 bildenden Sinterwerkstoffs wird über das Anschlussstück 5a Liquid aus dem Liquidspeicher 5 in den Liquidträger 6 gefördert, welches bei aktiviertem Heizelement 7 zur Durchführung eines Inhalationsvorgangs verdampft wird.

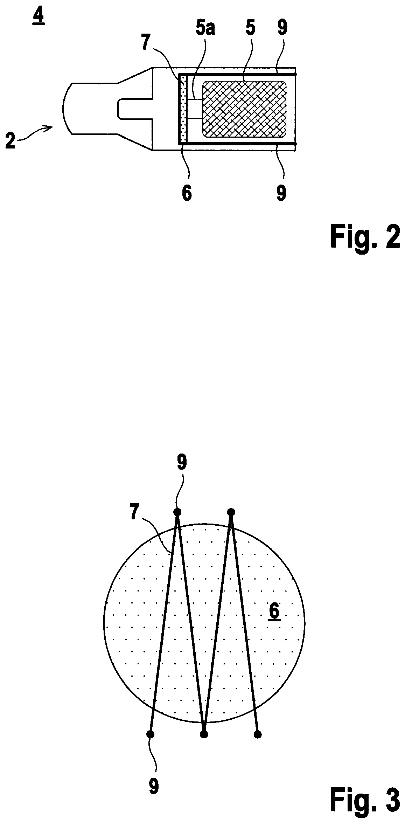

Figur 2 zeigt eine nicht zur Erfindung gehörige Variante des Verdampfers 4 gemäß Figur 1. Der Liquidspeicher 5 besteht analog zur Ausführungsform gemäß Figur 1 aus einem kreiszylinderischen Tank mit einem Anschlussstück 5a an einem längsseitigen Ende. Auf diesem Anschlussstück 5a sitzt wieder der Liquidträger 6 auf, der analog zur Ausführungsform gemäß Figur 1 von einem massiven Formteil bestehend aus einem Sinterwerkstoff gebildet ist. Anders als bei der Ausführungsform gemäß Figur 1 besteht der Liquidträger 6 gemäß Figur 2 aus einem plattenförmigen Formteil, der im vorliegenden Fall kreisscheibenförmig ausgebildet ist. Der so gebildete Liquidträger 6 ist koaxial zum Liquidspeicher 5 angeordnet. Auf der dem Liquidspeicher 5 abgewandten Stirnseite liegt das von einem Heizdraht gebildete Heizelement 7 auf, wie insbesondere Figur 3 zeigt. Anstelle eines Heizelements 7 kann analog zur Ausführungsform gemäß Figur 1 ein aus elektrisch leitfähigen Bändern oder aus einer elektrisch leitfähigen Beschichtung auf dem Liquidträger 6 ausgebildetes Heizelement 7 eingesetzt werden. Zum Anschluss des Heizelements 7 an die elektronische Baueinheit 8 sind wieder Anschlussklemmen 9 vorgesehen.

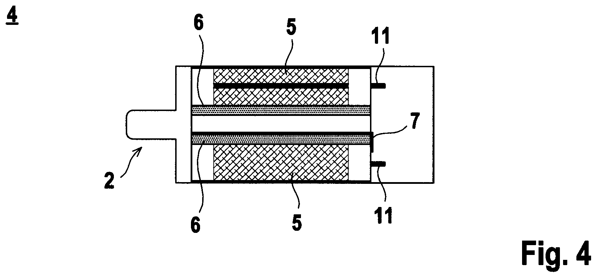

Figur 4 zeigt die erfindungsgemäße Ausbildung des Verdampfers 4 der elektronischen Zigarette gemäß Figur 1. In diesem Fall besteht der Liquidträger 6 aus einem rohrförmigen Formteil mit kreisförmigem Querschnitt bestehend aus Sinterwerkstoff. Die Längsachse des Formteils verläuft in der Längsachse des Gehäuses 3 der elektronischen Zigarette. Der wieder als Tank ausgebildete Liquidspeicher 5 weist eine kreiszylindrische Außenkontur auf. Weiterhin weist der Liquidspeicher 5 eine zentrale, entlang dessen Längsachse verlaufende Bohrung auf, in welcher der Liquidträger 6 geführt ist. Dabei liegt die äußere Mantelfläche des Liquidträgers 6 dicht an der inneren Mantelfläche des Liquidspeichers an. An seiner inneren Mantelfläche sind nicht dargestellte Anschlusselemente vorgesehen, über die Liquid durch die Kapillarwirkdung des Sinterwerkstoffs in den Liquidträger 6 gefördert wird.

Das wiederum von einem Heizdraht gebildete Heizelement 7 liegt an der inneren Mantelfläche des Liquidträgers 6, welche einen Hohlraum umschließt, an. Der Heizdraht ist über Kontaktstecker 11 an die elektronische Baueinheit 8 angeschlossen. Auch in diesem Fall können anstelle eines Heizdrahts zur Ausbildung des Heizelements 7 elektrisch leitfähige, bandförmige Materialien oder Beschichtungen eingesetzt werden.

### Bezugszeichenliste

- (1): Elektronische Zigarette
- (2): Mundstück
- (3): Gehäuse
- (4): Verdampfer
- (5): Liquidspeicher
- (5a): Anschlussstück
- (6): Liquidträger
- (7): Heizelement
- (8): Baueinheit
- (9): Anschlussklemmen
- (10): Batterie
- (11): Kontaktstecker

## Patentansprüche

1. Elektronische Zigarette (1) oder Pfeife mit einem Mundstück (2) und einem Verdampfer (4), welcher einen Liquidträger (6) und ein Heizelement (7), welches mit einem Aktivierungsmittel aktivierbar ist, umfasst, wobei das aktivierte Heizelement (7) im Liquidträger (6) vorhandenes Liquid verdampft, und wobei der Liquidträger (6) nur von einem aus einem Sinterwerkstoff bestehenden Formteil gebildet ist, wobei das Heizelement (7) direkt in Kontakt mit einer Oberfläche des Liquidträgers (6) ist, **dadurch gekennzeichnet, dass** der Liquidträger (6) rohrförmig, insbesondere als Hohlzylinder mit kreisförmigem Querschnitt ausgebildet ist, wobei das Heizelement (7) an der inneren Mantelfläche des rohrförmigen Liquidträgers (6) anliegt, und wobei der Liquidspeicher (5) den rohrförmigen Liquidträger (6) umschließt und an dessen äußerer Mantelfläche anliegt.

2. Elektronische Zigarette nach Anspruch 1, **dadurch gekennzeichnet, dass** diese einen Liquidspeicher (5) aufweist, aus welchem dem Liquidträger (6) Liquid zugeführt wird.

3. Elektronische Zigarette oder Pfeife nach Anspruch 1, **dadurch gekennzeichnet, dass** der Liquidträger (6) aus einem elektrisch nicht leitfähigem Sinterwerkstoff besteht.

4. Elektronische Zigarette oder Pfeife nach einem der Ansprüche 1 oder 2, **dadurch gekennzeichnet, dass** der Sinterwerkstoff von einer gesinterten Keramik gebildet ist.

5. Elektronische Zigarette oder Pfeife nach einem der Ansprüche 1 oder 2, **dadurch gekennzeichnet, dass** der Sinterwerkstoff von Borosilikatgläsern, Aluminiumoxid-Mullit, Cordierit, Siliziumnitrit, Siliziumcarbid, Tialit, Steatit, Porzellan, Zirkon oder ZrO₂ gebildet ist.

6. Elektronische Zigarette nach Anspruch 1, **dadurch gekennzeichnet, dass** der Liquidträger (6) aus einem porösen Festkörper besteht.

7. Elektronische Zigarette oder Pfeife nach einem der Ansprüche 1 bis 6, **dadurch gekennzeichnet, dass** das Heizelement (7) ein Heizdraht ist, oder aus bandförmigem elektrisch leitendem Material besteht, oder als leitfähige Beschichtung auf einer Oberfläche des Liquidträgers (6) ausgebildet ist.

## Claims

1. Electronic cigarette (1) or pipe with a mouthpiece (2) and an evaporator (4), which comprises a liquid carrier (6) and a heating element (7) activatable by activation means, wherein the activated heating element (7) evaporates liquid present in the liquid carrier (6), wherein the liquid carrier (6) is formed only by a shaped part consisting of a sintered material and wherein the heating element (7) is directly in contact with the surface of the liquid carrier (6), **characterised in that** the liquid carrier (6) is formed to be tubular, particularly as a hollow cylinder with circular cross-section, wherein the heating element (7) bears against the inner circumferential surface of the tubular liquid carrier (6) and wherein the liquid store (5) encloses the tubular liquid carrier (6) and bears against the outer circumferential surface thereof.

2. Electronic cigarette according to claim 1, **characterised in that** this comprises a liquid store (5) from which liquid is fed to the liquid carrier (6).

3. Electronic cigarette or pipe according to claim 1, **characterised in that** the liquid carrier (6) consists of an electrically non-conductive sintered material.

4. Electronic cigarette or pipe according to any one of claims 1 and 2, **characterised in that** the sintered material is formed by a sintered ceramic.

5. Electronic cigarette or pipe according to one of claims 1 and 2, **characterised in that** the sintered material is formed from borosilicate glasses, aluminium oxide mullite, cordierite, silicon nitrite, silicon carbide, tialite, steatite, porcelain, zircon or ZrO₂.

6. Electronic cigarette according to claim 1, **characterised in that** the liquid carrier (6) consists of a porous solid body.

7. Electronic cigarette or pipe according to any one of claims 1 to 6, **characterised in that** the heating element (7) is a hot wire or consists of strip-shaped electrically conductive material or is formed as a conductive coating on a surface of the liquid carrier (6).

## Revendications

1. Cigarette (1) ou pipe électronique équipée d'un embout buccal (2) et d'un vaporisateur (4), qui inclut un support (6) de liquide et un élément chauffant (7) pouvant être activé par un agent d'activation, sachant que l'élément chauffant activé (7) vaporise du liquide présent dans le support (6) de liquide, et que ledit support (6) de liquide est constitué uniquement d'une pièce moulée consistant en un matériau fritté, ledit élément chauffant (7) étant directement en contact avec une surface dudit support (6) de liquide, **caractérisée par le fait que** le support (6) de liquide est de réalisation tubulaire, notamment en tant que cylindre creux muni d'une section transversale circulaire, l'élément chauffant (7) étant en applique contre la surface intérieure de l'enveloppe du support tubulaire (6) de liquide, sachant que le réservoir (5) de liquide ceinture ledit support tubulaire (6) de liquide, et est en applique contre la surface extérieure de l'enveloppe de ce dernier.

2. Cigarette électronique selon la revendication 1, **caractérisée par le fait qu'**elle est pourvue d'un réservoir (5) de liquide, à partir duquel du liquide est délivré au support (6) de liquide.

3. Cigarette ou pipe électronique selon la revendication 1, **caractérisée par le fait que** le support (6) de liquide consiste en un matériau fritté électriquement non conducteur.

4. Cigarette ou pipe électronique selon l'une des revendications 1 ou 2, **caractérisée par le fait que** le matériau fritté est constitué d'une céramique frittée.

5. Cigarette ou pipe électronique selon l'une des revendications 1 ou 2, **caractérisée par le fait que** le matériau fritté est constitué de verres borosilicates, d'alumine-mullite, de cordiérite, de nitrure de silicium, de carbure de silicium, de tialite, de stéatite, de porcelaine, de zircon ou de ZrO₂.

6. Cigarette électronique selon la revendication 1, **caractérisée par le fait que** le support (6) de liquide consiste en un corps solide poreux.

7. Cigarette ou pipe électronique selon l'une des revendications 1 à 6, **caractérisée par le fait que** l'élément chauffant (7) est un fil métallique chauffant, ou consiste en un matériau en bandes électriquement conducteur, ou bien est réalisé sous la forme d'un revêtement conducteur sur une surface du support (6) de liquide.
